# EUROPÄISCHE PATENTSCHRIFT

(11) **EP 3 519 781 B1**
(45) Veröffentlichungstag und Bekanntmachung des Hinweises auf die Patenterteilung: **18.11.2020**
(21) Anmeldenummer: 17739875.7
(22) Anmeldetag: 14.07.2017
(51) Int. Cl.: G01G 17/04, A61M 5/14, G01N 35/08

(54) **VERTEILERKOPF FÜR EINE SAUGPUMPE**
DISTRIBUTOR HEAD FOR A SUCTION PUMP
TÊTE DE DISTRIBUTION POUR POMPE D'ASPIRATION

(30) Priorität: 27.09.2016 DE 102016118183
(43) Veröffentlichungstag der Anmeldung: 07.08.2019
(73) Patentinhaber: Sartorius Lab Instruments GmbH & Co. KG, 37079 Göttingen (DE)
(72) Erfinder: BODE, Jonas, 37170 Uslar (DE); STÄNDER, Malte, 37308 Heiligenstadt (DE)
(74) Vertreter: Vigand, Philippe
(86) Internationale Anmeldenummer: PCT/EP2017/000848
(87) Internationale Veröffentlichungsnummer: WO 2018/059723

(56) Entgegenhaltungen:
- WO-A1-88/00347

## Beschreibung

### Gebiet der Erfindung

Die Erfindung betrifft einen Verteilerkopf für eine Saugpumpe, umfassend
- ein Gehäuse,
- einen Pumpenanschluss zum Anlegen eines Unterdrucks und
- eine Mehrzahl an dem Gehäuse festgelegter, flüssigkeitsdicht mit dem Pumpenanschluss verbundener, parallel und beabstandet zueinander angeordneter Hohlnadeln.

### Stand der Technik

Aus der EP 2 759 816 B1 ist eine Prüfvorrichtung zum gravimetrischen Prüfen von Mehrkanalpipetten bekannt. Derartige Prüfvorrichtungen enthalten in einem Gehäuse eine Mehrzahl von versetzt zueinander angeordneten Wägezellen, deren Lastaufnehmer in einer geraden, horizontalen Reihe äquidistant angeordnet sind. Jeder Lastaufnehmer trägt ein schmales, zylindrisches Gefäß, in welches beim Prüfvorgang Flüssigkeit aus einer Pipette hinein pipettiert werden kann. Die Anordnung der einzelnen Lastaufnehmer und ihrer Pipettiergefäße ist der Anordnung der Pipettenspitzen der zu kalibrierenden Mehrkanalpipette angepasst. Auf diese Weise lassen sich sämtliche Pipettenkanäle gleichzeitig in die jeweils zugeordneten Pipettiergefäße hinein entleeren und das jeweilige Pipettiervolumen lässt sich gravimetrisch ermitteln. Vor jedem neuen Pipettiervorgang können die Wägezellen bedarfsweise tariert werden, sodass der jeweilige Vor-Füllstand der Gefäße die einzelnen Messungen nicht beeinflusst. Nach einer gewissen Anzahl von Prüfvorgängen ist es jedoch unerlässlich, die Pipettiergefäße zu entleeren. Die genannte Druckschrift offenbart hierzu die parallele Entleerung sämtlicher Pipettiergefäße mittels Absaugung durch eine Mehrkanal-Saugpumpe.

Derartige Saugpumpen umfassen typischerweise eine einzige, den erforderlichen Unterdruck erzeugende Pumpstation, die an den Pumpenanschluss eines gattungsgemäßen Verteilerkopfs angeschlossen ist. Der Verteilerkopf weist eine Mehrzahl an seinem Gehäuse festgelegter Hohlnadeln auf, deren Anordnung und Orientierung derjenigen der Pipettiergefäße der gravimetrischen Prüfvorrichtung angepasst sind. Die einzelnen Hohlnadeln sind flüssigkeitsdicht mit dem Pumpenanschluss verbunden. Üblicherweise ist hierzu eine Stammleitung mit dem Pumpenanschluss verbunden, von welcher einzelne Zweigleitungen zu den einzelnen Hohlnadeln abgehen. Denkbar ist selbstverständlich auch die individuelle Leitungsführung von jeder Hohlnadel zum gemeinsamen Pumpenanschluss. Zum Abpumpen wird der Verteilerkopf so positioniert, dass sämtliche Hohlnadeln bis zur gewünschten Eintauchtiefe in die Pipettiergefäße eingetaucht werden. Der von der Pumpstation erzeugte Unterdruck verteilt sich sodann auf sämtliche Saugkanäle, sodass die Flüssigkeit aus allen Pipettiergefäßen parallel abgesaugt wird.

Probleme können jedoch auftreten, wenn der Füllstand in einem Pipettiergefäß deutlich niedriger ist als in einem anderen Pipettiergefäß. Das nur gering befüllte Gefäß wird alsbald leer gesaugt sein, sodass die entsprechende Hohlnadel Luft zieht. Wird keine extrem starke und damit große, schwere und teure Pumpstation verwendet, führt dies zu einem Abriss der Flüssigkeitssäule im gemeinsamen Leitungsbereich, sodass die Absaugung insgesamt stoppt. Die höher befüllten Gefäße werden daher nicht vollständig abgesaugt. Wird beim weiteren Pipettieren jedoch fälschlicherweise davon ausgegangen, dass alle Gefäße leer sind, kann es zum Überlaufen der Gefäße und zur Beschädigung des empfindlichen Innenlebens der Prüfvorrichtung kommen.

Weiterer Stand der Technik ist Druckschrift WO88/00347.

### Aufgabenstellung

Es ist die Aufgabe der vorliegenden Erfindung, einen gattungsgemäßen Verteilerkopf für Saugpumpen derart weiterzubilden, dass eine zuverlässig gleichmäßige Absaugung auf sämtlichen Kanälen gewährleistet wird.

### Darlegung der Erfindung

Diese Aufgabe wird in Verbindung mit den Merkmalen des Oberbegriffs von Anspruch 1 gelöst.

Bevorzugte Ausführungsformen der Erfindung sind Gegenstand der abhängigen Patentansprüche.

Durch das jedem einzelnen Kanal zugeordnete Schaltventil, das selbstverständlich jeweils im kanalindividuellen Abschnitt des jeweiligen Verbindungspfades angeordnet zu sein hat, ist es möglich, die einzelnen Kanäle individuell und unabhängig voneinander zu sperren. Ist also eines der Pipettiergefäße vorzeitig geleert und zieht seine zugeordnete Hohlnadel folglich Luft, kann der entsprechende Saugkanal geschlossen werden, sodass sich der von der Pumpstation erzeugte Unterdruck nur noch auf die verbleibenden, offenen Kanäle verteilt, deren zugeordnete Gefäße folglich weiter durch Absaugung entleert werden. So kann durch sukzessives Abschalten jedes luftziehenden Kanals die gleichmäßige Entleerung aller Gefäße, d.h. ihre Entleerung bis zum selben Restfüllstand (was selbstverständlich auch die vollständige Leersaugung umfasst) sichergestellt werden.

Dabei kann, wie bei einer bevorzugten Ausführungsform der Erfindung, vorgesehen sein, dass die Hohlnadeln in ihrer Höhenlage individuell justierbar sind. Damit kann eine Anpassung an ggf. unterschiedliche Höhenlagen der Pipettiergefäße innerhalb der Prüfvorrichtung bewerkstelligt werden. Auch ist es damit möglich, unterschiedliche Restfüllstände für unterschiedliche Pipettiergefäße vorzugeben.

Um das Erkennen eines luftziehenden Kanals und damit seine gezielte Abschaltung zu erleichtern, ist erfindungsgemäß vorgesehen, dass jedem Schaltventil eine Prüfkammer zugeordnet ist, deren Innenraum Bestandteil des jeweiligen Verbindungspfades ist und deren Außenwandung ein das Gehäuse durchsetzendes Sichtfenster umfasst. Sobald ein Kanal Luft zieht, treten in ihm Blasen auf, die in Richtung Pumpstation wandern. Dabei durchlaufen sie auch die Prüfkammer, was vom Benutzer durch das Sichtfenster hindurch von außen detektiert werden kann. Durch die vorgegebene Zuordnung jedes Sichtfensters zu einem speziellen Schaltventil, ist das zur Abschaltung des Kanals zu schaltende Schaltventil leicht identifizierbar.

Die Prüfkammern sind erfindungsgemäß hohlnadelseitig des jeweils zugeordneten Schaltventils im jeweiligen Verbindungspfad angeordnet. Es hat sich nämlich herausgestellt, dass die Schaltventile - zumindest im geschlossenen Zustand - als Isolatoren für die sich in den Verbindungspfaden fortsetzenden, schnellen, von der Pumpstation erzeugten Druckschwankungen wirken. Diese bringen Luftblasen in der Prüfkammer zu einem durch das Sichtfenster erkennbaren Zittern. Durch die hohlnadelseitige Anordnung der Prüfkammern lässt sich daher anhand des Ruhens bzw. Zitterns der Luftblase ein Kanal mit (nach Luftziehen) bereits geschlossenem Ventil eindeutig von einem solchen mit offenem Ventil unterscheiden.

Alternativ zu der oben geschilderten, visuellen Blasendetektion und manuellen Abschaltung der einzelnen Kanäle durch einen menschlichen Benutzer ist alternativ auch eine Automatisierung möglich. Dem Fachmann sind unterschiedliche Verfahren zur Blasendetektion, beispielsweise optisch oder kapazitiv, bekannt. Diese lassen sich ohne Weiteres mit einer elektronischen Ansteuerung der Ventile koppeln.

Zu Beginn jedes Absaugungsvorgangs sollten alle Ventile geöffnet sein. Nur so kann sichergestellt werden, dass sämtliche Gefäße abgesaugt werden. Bleibt ein bei einem vorangehenden Absaugprozess einzeln geschlossenes Ventil beim nächsten Absaugvorgang versehentlich geschlossen, kann es bei nachfolgenden Pipettiervorgängen zum unerwünschten Überlauf kommen. Im Rahmen der oben erwähnten, Elektronik-basierten Automatisierung ist es unproblematisch, dies mit programmiertechnischen Mitteln sicherzustellen. Um auch rein manuell betätigte Ausführungsformen fehlerunanfällig zu gestalten, ist bei einer Weiterbildung der Erfindung vorgesehen, dass die Schaltventile als unterdrucksensitive Normally-Open-Ventile ausgebildet sind. Als Normally-Open-Ventil bezeichnet der Fachmann allgemein ein Ventil, welches im unbetätigten Zustand offen ist. "Unbetätigt" kann sich dabei sowohl auf eine nicht vorhandene, mechanische Betätigung als auch auf eine fehlende Bestromung eines elektrischen Ventils beziehen. "Unterdrucksensitiv" bedeutet in diesem Zusammenhang, dass sich der nicht betätigte, d.h. offene Zustand des Ventils automatisch einstellt, wenn kein Unterdruck am Ventil anliegt. Dem Fachmann sind verschiedene Möglichkeiten bekannt, dies sowohl bei elektrischen als auch bei rein mechanischen Ventilen zu realisieren.

Das Einführen der parallelen Hohlnadeln in die Pipettiergefäße hat vorsichtig zu erfolgen, um ein Anschlagen der Nadeln an der Gefäßwand und dadurch evtl. eine Beschädigung der Wägezellen zu vermeiden. Bei einer Weiterbildung der Erfindung ist daher vorgesehen, dass der Verteilerkopf mittels wenigstens einer parallel zu den Hohlnadeln am Gehäuse angeordneten Teleskopstange geführt verfahrbar ist. Die Teleskopstange kann mittelbar oder unmittelbar auf der Pipettenprüfvorrichtung aufgesetzt werden, sodass die Bewegung des Verteilerkopfs beim Absenken der Hohlnadeln in die Pipettiergefäße geführt erfolgt. Dabei kann die Teleskopstange ausschließlich der Führung der Bewegung dienen. Denkbar ist jedoch auch, dass durch einen in der Teleskopstange integrierten Antrieb, beispielsweise einen Spindeltrieb, eine motorische Verfahrbarkeit realisiert wird. Um auch eine Rotation um die Teleskopstange zu verhindern, sind bevorzugt zwei oder mehr Teleskopstangen zur Bewegungsführung vorgesehen. Diese können unmittelbar auf der Prüfvorrichtung aufgesetzt oder, bevorzugt, durch eine Rahmenplatte verbunden sein, die ihrerseits positionstreu auf der Prüfvorrichtung aufgesetzt werden kann. Die Positionstreue kann insbesondere durch korrespondierende Formschlusskonturen an Rahmenplatte und Prüfvorrichtung gewährleistet werden.

Die nach Absaugung zu erreichende Restfüllhöhe kann anwendungsspezifisch verschieden sein. Insbesondere kann ein vollständiges Absaugen vorgesehen sein, wenn keine weiteren Prüfdurchgänge mehr beabsichtigt sind. Verschiedene Prüfnormen sehen jedoch einen Mindestfüllstand der Pipettiergefäße während der Prüfpipettierung vor. Um solch unterschiedlichen Anforderungen gerecht zu werden, ist bei einer Weiterbildung der Erfindung vorgesehen, dass der Verteilerkopf einen seine Verfahrstrecke begrenzenden, verstellbaren Anschlag aufweist. Besonders bevorzugt ist dieser Anschlag in der Teleskopstange integriert.

Um Erschütterungen zu vermeiden, ist die Teleskopstange günstigerweise mit einem Dämpfungsmechanismus versehen. Es kann sich hierbei um eine Dämpferhydraulik handeln, wie sie beispielsweise von Industrie-Stoßdämpfern bekannt ist. Dies erlaubt ein ruckloses Einfahren des Verteilerkopfes in seine Absaugstellung.

Alternativ oder zusätzlich zu einer Teleskopstange kann selbstverständlich auch eine starre Führungsstange Anwendung finden.

Weitere Merkmale und Vorteile der Erfindung ergeben sich aus der nachfolgenden speziellen Beschreibung und den Zeichnungen.

### Kurzbeschreibung der Zeichnungen

Es zeigen:
- Figur 1:: eine vereinfachte, perspektivische Darstellung eines erfindungsgemäßen Verteilerkopfes und
- Figur 2:: eine schematische Darstellung der Leitungsanordnung im Verteilerkopf von Figur 1.

### Ausführliche Beschreibung bevorzugter Ausführungsformen

Gleiche Bezugszeichen in den Figuren deuten auf gleiche oder analoge Elemente hin.

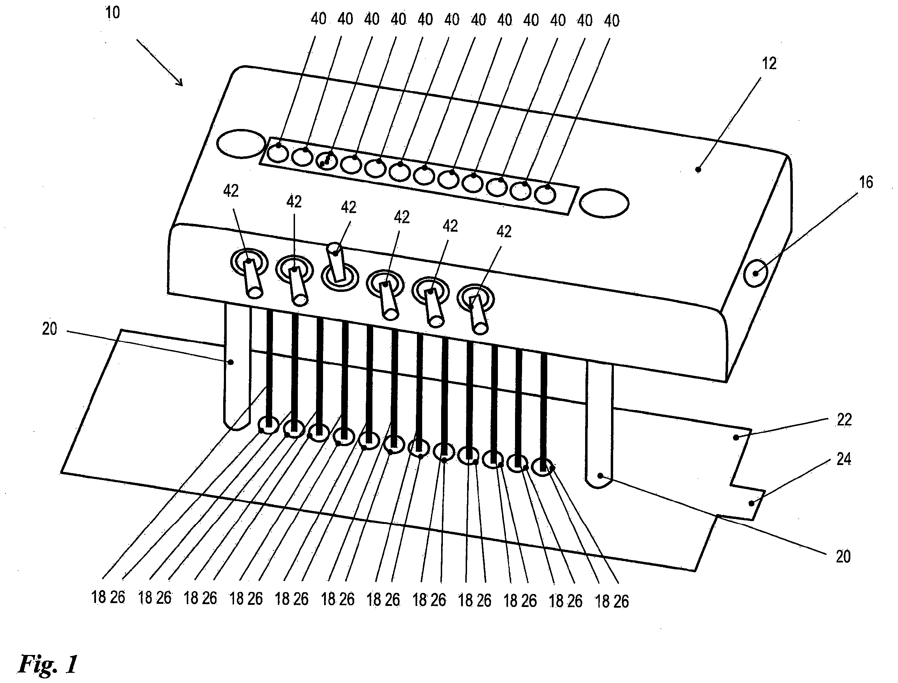

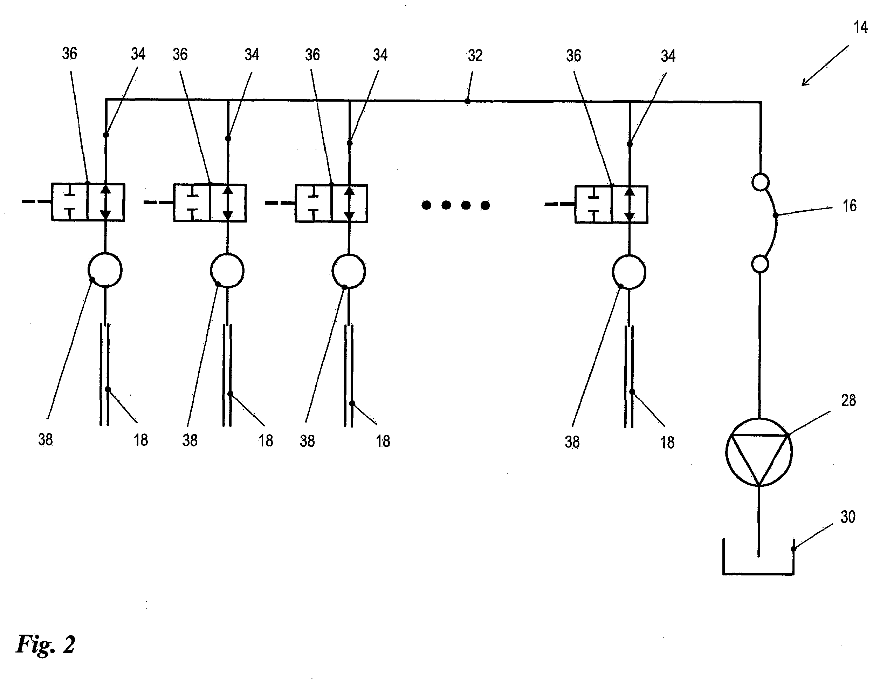

Figur 1 zeigt in vereinfachter, perspektivischer Darstellung eine bevorzugte Ausführungsform eines erfindungsgemäßen Verteilerkopfes 10. Der Verteilerkopf 10 umfasst ein Gehäuse 12, in dem eine Leitungsanordnung 14 vorgesehen ist, wie sie in schematischer Darstellung in Figur 2 wiedergegeben ist. Die Leitungsanordnung 14 verbindet innerhalb des Gehäuses einen Pumpenanschluss 16 mit einer Mehrzahl von Hohlnadeln 18. Bei der dargestellten Ausführungsform sind zwölf Hohlnadeln vorgesehen, deren konkrete Anzahl jedoch nicht erfindungswesentlich ist. Die Hohlnadeln 18 sind parallel zueinander ausgerichtet und in einer geraden, äquidistanten Reihe am Gehäuse 12 festgelegt.

Das Gehäuse ist über zwei Teleskopstangen 20 mit einer Positionierplatte 22 verbunden, welche sich exakt auf einer nicht dargestellten gravimetrischen Prüfvorrichtung für Mehrkanalpipetten positionieren lässt. Die Positionstreue wird dabei über Formschlusskonturen 24 gewährleistet, die an der Prüfvorrichtung korrespondierende Konturen finden. Die Positionierung der Positionierplatte 22 auf der Prüfvorrichtung erfolgt so, dass die Hohlnadeln 18 exakt über den abzusaugenden Pipettiergefäßen positioniert werden. Durch Kompression der Teleskopstangen 20 können die Hohlnadeln 18 senkrecht und berührungslos in die Pipettiergefäße abgesenkt werden, wobei sie entsprechende Durchbrechungen 26 in der Positionierplatte 22 durchgreifen. Die Eintauchtiefe kann dabei durch einen in Figur 1 nicht im Detail dargestellten, verstellbaren Anschlag justiert werden.

Der Pumpenanschluss 16 dient dem flüssigkeitsdichten Anschluss einer Pumpstation 28, mittels welcher ein Unterdruck erzeugt werden kann, sodass Flüssigkeit aus den Pipettiergefäßen über die Hohlnadeln 18 und die Leitungsanordnung 14 in einen Tank 30 abgesaugt werden kann.

Wie in Figur 2 erkennbar, umfasst die Leitungsanordnung 14 eine Stammleitung 32 von der für jede Hohlnadel 18 eine Zweigleitung 34 abgeht. Die Zweigleitungen 34 passieren jeweils ein als unterdrucksensitives Normally-Open-Ventil ausgestaltetes Schaltventil 36 und münden in die Hohlnadeln 18. Hohlnadelseitig der Schaltventile 36 ist in jeder Zweigleitung 34 eine Prüfkammer 38 angeordnet, deren eine Kammerwandung als Sichtfenster 40 im Deckel des Gehäuses 12 ausgebildet ist.

Nach Positionierung der Hohlnadeln 18 in den abzusaugenden Pipettiergefäßen wird die Pumpstation 28 eingeschaltet. Durch die offenen Ventile 36 wird Flüssigkeit aus den Pipettiergefäßen in den Tank 30 gepumpt. Fällt der Flüssigkeitsspiegel in einem Pipettiergerät dabei unter das freie Ende der zugeordneten Hohlnadel 18, zieht diese Hohlnadel Luft. Entsprechend bilden sich Blasen in der Prüfkammer 38, die durch das entsprechende Sichtfenster 40 für den Benutzer detektierbar ist. In Figur 1 sind im dritten Fenster von links derartige Blasen dargestellt. Um eine weitere Absaugung der übrigen Pipettiergefäße zu ermöglichen, kann mittels eines Schalters 42, der jedem Schaltventil 36 zugeordnet ist, die entsprechende Zweigleitung 34 gesperrt werden. Das Leitungssystem 14 zieht dann insgesamt keine Luft mehr, sodass weiter Flüssigkeit aus den Pipettiergefäßen abgesaugt wird, bis der Flüssigkeitspegel eines weiteren Gefäßes unter die Spitze der zugeordneten Hohlnadel 18 fällt. Bei der Ausführungsform von Figur 1 ist der dritte Schalter 42 von links der Prüfkammer 38, die mittels des dritten Sichtfensters 40 von links einsehbar ist, zugeordnet und in bereits betätigter Stellung dargestellt. Bei der gezeigten Ausführungsform sind die sechs sichtbaren Schalter 42 den sechs linken Hohlnadeln 18 zugeordnet, wohingegen die den sechs rechten Hohlnadeln 18 zugeordneten Schaltventile 34 mittels Schaltern auf der nicht sichtbaren, gegenüberliegenden Gehäuseseite betätigt werden.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben. Insbesondere ist eine Automatisierung des oben erläuterten Absaugverfahrens möglich, wobei sowohl die Schaltung der Ventile als auch die Blasendetektion einzeln oder gemeinsam automatisierbar sind. Obgleich der erfindungsgemäße Verteilerkopf hier vorwiegend im Kontext eines Ausführungsbeispiels als separates Modul beschrieben wurde, erstreckt sich die Erfindung selbstverständlich auch auf solche Ausführungsformen, bei denen der Verteilerkopf integrierter Bestandteil einer komplexeren Anordnung ist.

### Bezugszeichenliste

- 10: Verteilerkopf
- 12: Gehäuse
- 14: Leitungsanordnung
- 16: Pumpenanschluss
- 18: Hohlnadel
- 20: Teleskopstange
- 22: Positionierplatte
- 24: Formschlusskontur
- 26: Durchbruch in 22
- 28: Pumpstation
- 30: Tank
- 32: Stammleitung
- 34: Zweigleitung
- 36: Schaltventil
- 38: Prüfkammer
- 40: Sichtfenster
- 42: Schalter

## Patentansprüche

1. Verteilerkopf für eine Saugpumpe, umfassend
- ein Gehäuse (12),
- einen Pumpenanschluss (16) zum Anlegen eines Unterdrucks und
- eine Mehrzahl flüssigkeitsdicht mit dem Pumpenanschluss (16) verbundener und beabstandet zueinander angeordneter Hohlnadeln (18),
wobei die einzelnen Verbindungspfade (34) der Hohlnadeln (18) zu dem Pumpenanschluss (16) jeweils ein unabhängig betätigbares Schaltventil (36) aufweisen,
**dadurch gekennzeichnet,**
**dass** die Hohlnadeln (18) am Gehäuse (12) festgelegt und parallel zueinander angeordnet sind und jedem Schaltventil (36) eine Prüfkammer (38) zugeordnet ist, deren Innenraum Bestandteil des jeweiligen Verbindungspfades (34) ist und deren Außenwandung ein das Gehäuse (12) durchsetzendes Sichtfenster (40) umfasst, wobei die Prüfkammern (38) hohlnadelseitig des jeweils zugeordneten Schaltventils (36) im jeweiligen Verbindungspfad (34) angeordnet sind.

2. Verteilerkopf nach Anspruch 1,
**dadurch gekennzeichnet,**
**dass** die Hohlnadeln in ihrer Höhenlage individuell justierbar sind.

3. Verteilerkopf nach einem der vorangehenden Ansprüche,
**dadurch gekennzeichnet,**
**dass** die Schaltventile (36) als unterdrucksensitive Normally-Open-Ventile ausgebildet sind.

4. Verteilerkopf nach einem der vorangehenden Ansprüche,
**dadurch gekennzeichnet,**
**dass** er mittels wenigstens einer parallel zu den Hohlnadeln (18) am Gehäuse (12) angeordneten Teleskopstange (20) geführt verfahrbar ist.

5. Verteilerkopf nach Anspruch 4,
**dadurch gekennzeichnet,**
**dass** er einen seine Verfahrstrecke begrenzenden, verstellbaren Anschlag aufweist.

6. Verteilerkopf nach einem der Ansprüche 4 bis 5,
**dadurch gekennzeichnet,**
**dass** die Teleskopstange (20) einen Dämpfungsmechanismus umfasst.

## Claims

1. A distributor head for a suction pump, comprising
- a housing (12)
- a pump connection (16) for applying a negative pressure and
- a plurality of hollow needles (18) connected in fluid-tight manner to the pump connection (16) and arranged spaced apart to each other,
wherein the individual connection paths (34) of the hollow needles (18) to the pump connection (16) each have an independently operable switching valve (36),
**characterized in that**
the hollow needles (18) are arranged fixed on the housing (12) and parallel to one another and each switching valve (36) has a test chamber (38) assigned to it, whose interior is a component of the respective connection path (34) and whose outer wall comprises a viewing window (40) penetrating the housing (12),
wherein the test chambers (38) are arranged on the hollow needle side of the respective assigned switching valve (36) in the respective connection path (34).

2. The distributor head according to claim 1,
**characterized in that**
the hollow needles are individually adjustable in their altitude.

3. The distributor head according to any of the preceding claims,
**characterized in that**
the switching valves (36) are embodied as negative pressure-sensitive normally open valves.

4. The distributor head according to any of the preceding claims,
**characterized in that**
it can be moved in guided manner by means of a telescopic rod (20) arranged parallel to the hollow needles (18) on the housing (12).

5. The distributor head according to claim 4,
**characterized in that**
it has an adjustable stop limiting its travel path.

6. The distributor head according to any of claims 4 to 5,
**characterized in that**
the telescopic rod (20) comprises a damping mechanism.

## Revendications

1. Tête de distribution pour une pompe d'aspiration, comprenant
- un boîtier (12),
- un raccord de pompe (16) pour l'application d'une pression négative et
- une pluralité d'aiguilles creuses (18) reliées au raccord de pompe (16) de manière étanche au fluide et disposées à distance les unes des autre,
dans laquelle les différents trajets de liaison (34) des aiguilles creuses (18) au raccord de pompe (16) présentent respectivement une soupape de commande (36) pouvant être actionnée de façon indépendante,
**caractérisée en ce**
**que** les aiguilles creuses (18) sont fixées sur le boîtier (12) et sont disposées parallèlement les unes aux autres et une chambre de contrôle (38), dont l'espace intérieur fait partie du trajet de liaison (34) respectif et dont la paroi extérieure comprend une fenêtre d'inspection (40) traversant le boîtier (12), est associée à chaque soupape de commande (36),
dans laquelle les chambres de contrôle (38) sont disposées dans le trajet de liaison (34) respectif côté aiguille creuse de la soupape de commande (36) respectivement associée.

2. Tête de distribution selon la revendication 1,
**caractérisée en ce**
**que** les aiguilles creuses peuvent être alignées individuellement dans leur position en hauteur.

3. Tête de distribution selon l'une quelconque des revendications précédentes,
**caractérisée en ce**
**que** les soupapes de commande (36) sont réalisées sous la forme de soupapes normalement ouvertes sensibles à la pression négative.

4. Tête de distribution selon l'une quelconque des revendications précédentes,
**caractérisée en ce**
**qu'**elle peut se déplacer de manière guidée au moyen d'au moins une tige télescopique (20) disposée sur le boîtier (12) parallèlement aux aiguilles creuses (18).

5. Tête de distribution selon la revendication 4,
**caractérisée en ce**
**qu'**elle présente une butée réglable, délimitant son parcours de déplacement.

6. Tête de distribution selon l'une quelconque des revendications 4 à 5,
**caractérisée en ce**
**que** la tige télescopique (20) comprend un mécanisme d'amortissement.
